(19) **Europäisches Patentamt** / **European Patent Office** / **Office européen des brevets**

(11) **EP 4 332 082 B1**

(12) **EUROPEAN PATENT SPECIFICATION**

(45) Date of publication and mention
of the grant of the patent:
**17.06.2026 Bulletin 2026/25**

(21) Application number: **22795829.5**

(22) Date of filing: **27.04.2022**

(51) International Patent Classification (IPC):
**C07C 67/62** *(2006.01)* **C07C 69/54** *(2006.01)*
**C08F 220/12** *(2006.01)* **C08K 5/08** *(2006.01)*
**C08K 5/13** *(2006.01)* **C08K 5/17** *(2006.01)*
**C08K 5/32** *(2006.01)* **C08K 5/34** *(2006.01)*
**C08K 5/526** *(2006.01)*

(52) Cooperative Patent Classification (CPC):
(C-Sets available)
**C07C 67/62; C08F 220/12** (Cont.)

(86) International application number:
**PCT/JP2022/019007**

(87) International publication number:
**WO 2022/230916 (03.11.2022 Gazette 2022/44)**

(54) **METHYL-METHACRYLATE-CONTAINING COMPOSITION AND METHOD FOR PRODUCING METHYL METHACRYLATE POLYMER**

METHYLMETHACRYLATHALTIGE ZUSAMMENSETZUNG UND VERFAHREN ZUR HERSTELLUNG EINES METHYLMETHACRYLATPOLYMERS

COMPOSITION À TENEUR EN MÉTHACRYLATE DE MÉTHYLE, ET PROCÉDÉ DE FABRICATION DE POLYMÈRE DE MÉTHACRYLATE DE MÉTHYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2021 JP 2021075628**

(43) Date of publication of application:
**06.03.2024 Bulletin 2024/10**

(73) Proprietor: **Mitsubishi Chemical Corporation**
**Chiyoda-ku**
**Tokyo 100-8251 (JP)**

(72) Inventors:
• **KURIHARA, Yu**
**Tokyo 100-8251 (JP)**

• **KATO, Yuki**
**Tokyo 100-8251 (JP)**
• **SUZUKI, Tatsuya**
**Tokyo 100-8251 (JP)**
• **NINOMIYA, Wataru**
**Tokyo 100-8251 (JP)**

(74) Representative: **Hoffmann Eitle**
**Patent- und Rechtsanwälte PartmbB**
**Arabellastraße 30**
**81925 München (DE)**

(56) References cited:
WO-A1-2015/119233    JP-A- 2001 151 817
JP-A- 2007 045 803    JP-A- 2017 515 878
JP-A- 2020 524 210    JP-A- 2022 077 496
JP-A- S61 126 050    US-A- 5 877 344
US-B1- 6 448 354

(52) Cooperative Patent Classification (CPC): (Cont.)

   C-Sets
**C07C 67/62, C07C 69/54;**
**C08F 220/12, C08F 220/44;**
**C08K 5/08, C08L 33/18;**

**C08K 5/13, C08L 33/18;**
**C08K 5/17, C08L 33/18;**
**C08K 5/32, C08L 33/18;**
**C08K 5/3435, C08L 33/18;**
**C08K 5/45, C08L 33/18;**
**C08K 5/526, C08L 33/18**

**Description**

TECHNICAL FIELD

The present invention relates to a methyl methacrylate-containing composition and a method of producing methyl methacrylate polymer.

BACKGROUND ART

Methyl methacrylate (hereinafter also referred to as "MMA") is known to be an extremely useful substance used as a raw material of various applications and types of polymers. For example, polymethyl methacrylate, which is a homopolymer of methyl methacrylate, is used in signboards, lighting equipment, automotive parts, construction-related materials, light guiding panels for flat displays, light diffusion plates, and the like, taking advantage of its excellent transparency, weather resistance, and other properties. In addition, copolymers of methyl methacrylate and other monomers are used in paints, adhesives, resin modifier, artificial marble, latices for paper, and the like. Various methods have been developed for industrially producing methyl methacrylate, and for example, the acetone cyanohydrin (ACH) method, the new acetone cyanohydrin (new ACH) method, the C4 direct oxidation method, the direct methyl esterification method, the ethylene method, and the new ethylene method are known (Non-Patent Document 1). In these production methods, methyl methacrylate of a quality suitable for the intended use is obtained by carrying out purification such as distillation to remove unreacted raw materials and by-products contained in the produced methyl methacrylate.

Since methyl methacrylate has a tendency to polymerize, it is known that the quality of methyl methacrylate is maintained by adding a polymerization inhibitor when producing methyl methacrylate or storing produced methyl methacrylate (Non-Patent Document 2). For example, Patent Document 1 describes that methyl ether of hydroquinone (MEHQ) is particularly preferable among various polymerization inhibitors. Patent Document 2 describes that N,N'-dialkyl-p-phenylenediamine and N-oxyl are preferable among various polymerization inhibitors. Patent Document 3 describes distillation of methyl methacrylate in the presence of a phenol polymerization inhibitor. Patent Document 4 describes use of a diphenylamine derivative as a polymerization inhibitor. Patent Document 5 describes use of a benzene triamine derivative as a polymerization inhibitor.

Patent Document 6 discloses the use of a synergistic mixture of two nitroxide compounds for inhibiting polymerization of ethylenically unsaturated acrylates.

PRIOR ART DOCUMENTS

PATENT DOCUMENTS

Patent Document 1: JP 2004-155757 A
Patent Document 2: JP 2005-502695 A
Patent Document 3: JP H10-504553 A
Patent Document 4: JP 2002-533309 A
Patent Document 5: JP 2002-513034 A
Patent Document 6: US 5 877 344 A

NON-PATENT DOCUMENTS

Non-Patent Document 1: Toru Kuroda, "Development of Catalyst for Producing Methyl Methacrylate", Catalysts & Catalysis, 45 (5), 366-371, 2003, Catalysis Society of Japan
Non-Patent Document 2: Takayuki Otsu, "On the Functions of Polymerization Inhibitors", Synthetic Organic Chemistry, 33 (8), 634-640, 1975, The Society of Synthetic Organic Chemistry, Japan

SUMMARY OF THE INVENTION

PROBLEMS TO BE SOLVED BY THE INVENTION

However, even in cases where polymerization inhibitors as described above are added, the quality of methyl methacrylate may deteriorate during storage. In view of the above circumstances, the purpose of the present invention is to provide a methyl methacrylate-containing composition with high quality stability during storage.

MEANS FOR SOLVING THE PROBLEMS

In order to achieve the above purposes, the present inventors have intensively studied. As a result, they found that, in methyl methacrylate with the quality deteriorated during storage, the concentration of methyl methacrylate is reduced, and methyl methacrylate dimers and methyl pyruvate are generated. The presence of methyl methacrylate dimers in methyl methacrylate may change the structure of methyl methacrylate polymer obtained by polymerization to adversely affect its physical properties. Furthermore, the presence of methyl pyruvate in methyl methacrylate causes coloration of methyl methacrylate polymer obtained by polymerization. The present inventors have found that inclusion of a nitrile compound represented by a specific structural formula in a methyl methacrylate-containing composition results in improved quality stability during storage, and reduced generation of methyl methacrylate dimers and methyl pyruvate, thereby completing the present invention.

Accordingly, the present invention provides the following [1] to [16]:

[1]: A methyl methacrylate-containing composition, comprising:

methyl methacrylate;
a nitrile compound represented by the following Formula (1); and
a polymerization inhibitor (component B),
wherein the concentration of methyl methacrylate is from 99 to 99.99% by mass,

$$R-C{\equiv}N \qquad (1)$$

wherein, in Formula (1) above, R represents a $C_{1-5}$ chain or cyclic alkyl group, a $C_{2-5}$ chain or cyclic alkenyl group, or a $C_{1-12}$ aryl or heteroaryl group, which may further have a substituent.

[2]: The methyl methacrylate-containing composition according to [1], wherein when the concentration of the component A is MA ($\mu$mol/L), and the concentration of the component B is MB ($\mu$mol/L), MB/MA is from 0.003 or more.
[3]: The methyl methacrylate-containing composition according to [1] or [2], wherein when the concentration of the component A is MA ($\mu$mol/L), MA is from 1 to 20,000 $\mu$mol/L.
[4]: The methyl methacrylate-containing composition according to [3], wherein the MA is from 10 to 15,000 $\mu$mol/L.
[5]: The methyl methacrylate-containing composition according to any of [1] to [4], wherein when the concentration of the component B is MB ($\mu$mol/L), MB is from 1 to 5,000 $\mu$mol/L.
[6]: The methyl methacrylate-containing composition according to [5], wherein the MB is from 10 to 2,500 $\mu$mol/L.
[7]: The methyl methacrylate-containing composition according to any of [1] to [6], wherein the molecular weight of the component A is 1,000 or less.
[8]: The methyl methacrylate-containing composition according to any of [1] to [7], wherein the component B is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound.
[9]: The methyl methacrylate-containing composition according to any of [1] to [8], wherein the component B is at least one polymerization inhibitor selected from the group consisting of a phenol compound, an N-oxyl compound, an amine compound, a phosphorus-containing compound, and a sulfur-containing compound.
[10]: The methyl methacrylate-containing composition according to any of [1] to [9], wherein the component B is at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine.
[11]: The methyl methacrylate-containing composition according to any of [1] to [10], wherein the concentration of methyl methacrylate is from 99.8 to 99.99% by mass.
[12]: The methyl methacrylate-containing composition according to any of [1] to [11], wherein the composition does not contain diacetyl, or the concentration of diacetyl contained is 55 $\mu$mol/L or less.
[13]: The methyl methacrylate-containing composition according to any of [1] to [12], wherein in the Formula (1), R is a $C_{1-3}$ chain or cyclic alkyl group, a $C_{2-3}$ chain or cyclic alkenyl group, or a $C_{6-8}$ aryl or heteroaryl group.
[14]: The methyl methacrylate-containing composition according to any of [1] to [13], wherein in the Formula (1), R is a methyl group, an ethyl group, a vinyl group, an isopropenyl group, or a phenyl group.

[15]: A method of producing a methyl methacrylate polymer, comprising a step of polymerizing a polymeric composition comprising a methyl methacrylate-containing composition according to any of [1] to [14].

[16]: The method of producing a methyl methacrylate polymer according to [15], wherein the polymeric composition comprises a monomer that can be copolymerized with methyl methacrylate.

EFFECT OF THE INVENTION

According to the present invention, a methyl methacrylate-containing composition having high quality stability with reduced generation of methyl methacrylate dimers and methyl pyruvate during storage can be provided.

DETAILED DESCRIPTION OF THE INVENTION

Embodiments according to the present invention are described below, but the present invention is not limited to them.

As used herein, any numerical value range indicated by the term "to" means any range including the numerical values described before and after the term "to" as the lower and upper limit values, respectively, and "A to B" means A or more and B or less.

[Methyl Methacrylate-containing Composition]

A methyl methacrylate-containing composition according to the present invention comprises methyl methacrylate, a nitrile compound represented by the following Formula (1) (component A), and a polymerization inhibitor (component B), wherein the concentration of methyl methacrylate is from 99 to 99.99% by mass.

$$R-C{\equiv}N \qquad (1)$$

In Formula (1) above, R represents a $C_{1-5}$ alkyl group, a $C_{2-5}$ alkenyl group, or a $C_{1-12}$ aryl group, which may further have a substituent.

The methyl methacrylate-containing composition may also comprise other compound (component C) or water as long as the concentration of methyl methacrylate of from 99 to 99.99% by mass is satisfied. Each item is described below in detail.

(Component A)

The methyl methacrylate-containing composition according to the present invention comprises a nitrile compound represented by the Formula (1) (component A). When the component A is included in the methyl methacrylate-containing composition, the generation of methyl methacrylate dimers and methyl pyruvate during storage of the methyl methacrylate-containing composition can be repressed. The reason for this is presumed as follows.

The component B described later traps radicals generated in methyl methacrylate, thereby reducing the dimerization reaction of methyl methacrylate due to the radical mechanism. However, the dimerization reaction of methyl methacrylate also proceeds due to the cation mechanism in the presence of an acid catalyst. It is considered that the component A that is the nitrile compound is weakly basic, and thus traps an acidic substance that is present in a trace amount in the methyl methacrylate composition to inhibit its function as an acid catalyst, thereby enabling reducing the dimerization reaction of methyl methacrylate. Thus, it is considered that the component A and the component B inhibit the methyl methacrylate dimerization reaction via different mechanisms, and when they coexist, the generation of methyl methacrylate dimers can be efficiently repressed. The hydrolysis of methyl methacrylate proceeds under basic conditions. The nitrile compound is weakly basic, and thus does not have strong basicity enough to cause hydrolysis of methyl methacrylate. Therefore, the component A is effective as a basic substance that traps acidic substances present in trace amounts.

On the other hand, methyl pyruvate is generated due to oxidation of methyl methacrylate with hydroxy radicals and oxygen molecules. The component B can trap hydroxy radicals, while the component A can trap a radical intermediate generated due to the reaction of a hydroxy radical and methyl methacrylate and convert the intermediate back to methyl methacrylate. Thus, it is considered that when the component A and the component B coexist, the generation of methyl pyruvate can be efficiently repressed.

The molecular weight of the component A is preferably 1,000 or less. When the molecular weight is 1,000 or less, the number of cyano groups per unit mass in the component A can be increased, so that the effect of the present invention can be obtained with less mass. The molecular weight of the component A is more preferably 800 or less, still more preferably 600 or less, and particularly preferably 400 or less.

R in the Formula (1) represents a $C_{1-5}$ alkyl group, a $C_{2-5}$ alkenyl group, or a $C_{1-12}$ aryl group, which may further have a substituent.

In cases where R satisfies the conditions described above, the weak basicity of the component A, and the reactivity with an acidic substance or a radical are maintained, so that the effect of the present invention can be obtained. R is a highly stable substituent, and thus can prevent the component A from being changed into other compounds during storage. R is preferably a $C_{1-3}$ alkyl group, a $C_{2-3}$ alkenyl group, or a $C_{6-8}$ aryl group. R is more preferably a methyl group, an ethyl group, a vinyl group, an isopropenyl group, or a phenyl group.

The alkyl group is a chain (linear or branched) alkyl group or a cyclic alkyl group. Examples of the chain alkyl group include a methyl group, an ethyl group, an n-propyl group, an isopropyl group, an n-butyl group, an isobutyl group, an s-butyl group, a t-butyl group, an n-pentyl group, and an isopentyl group. Among them, a methyl group, an ethyl group, an n-propyl group, or an isopropyl group is preferable, and a methyl group or an ethyl group is more preferable. Examples of the cyclic alkyl group include a cyclopropyl group, a cyclobutyl group, and a cyclopentyl group.

The alkenyl group is a chain (linear or branched) alkenyl group or a cyclic alkenyl group. Examples of the chain alkenyl group include a vinyl group, a 1-propenyl group, an isopropenyl group, a 2-butenyl group, a 1,3-butadienyl group, and a 2-pentenyl group. Among them, a vinyl group, a 1-propenyl group, or an isopropenyl group is preferable, and a vinyl group or an isopropenyl group is more preferable. Examples of the cyclic alkenyl group include a cyclopropenyl group, a cyclobutenyl group, and a cyclopentenyl group.

The aryl group includes a heteroaryl group containing oxygen, nitrogen, sulfur, or the like. Examples of the aryl group include a phenyl group, a mesityl group, a naphthyl group, a 2-methylphenyl group, a 3-methylphenyl group, a 4-methylphenyl group, a 2,3-dimethylphenyl group, a 2,4-dimethylphenyl group, a 2,5-dimethylphenyl group, a 2,6-dimethylphenyl group, a 2-ethylphenyl group, an isoxazolyl group, an isothiazolyl group, an imidazolyl group, an oxazolyl group, a thiazolyl group, a thiadiazolyl group, a thienyl group, a triazolyl group, a tetrazolyl group, a pyridyl group, a pyrazinyl group, a pyrimidinyl group, a pyridazinyl group, a pyrazolyl group, a pyrrolyl group, a furyl group, a furazanyl group, an isoquinolyl group, an isoindolyl group, an indolyl group, a quinolyl group, a pyridothiazolyl group, a benzimidazolyl group, a benzoxazolyl group, a benzothiazolyl group, a benzotriazolyl group, a benzofuranyl group, an imidazopyridinyl group, a triazopyridinyl group, and a purinyl group.

When R is an alkyl, alkenyl, or aryl group having a substituent, examples of the substituent include an alkyl group, an alkenyl group, an aryl group, a hydroxy group, an alkoxy group, an amino group, a monovalent group containing a carbonyl group, an alkylthio group, and an arylthio group. Among them, a hydroxy group, an alkoxy group, an amino group, a monovalent group containing a carbonyl group, or an alkylthio group is preferable, and a hydroxy group, a methoxy group, an amino group, an acetyl group, or a methylthio group is more preferable. The molecular weight of the substituent is preferably 200 or less, more preferably 100 or less, and still more preferably 50 or less. It is noted that the number of carbon atoms in R is the number including carbon atoms in such a substituent contained in an alkyl group, an alkenyl group, or an aryl group. For example, in a case of 4-(methylthio)benzonitrile, R is a 4-(methylthio)phenyl group, and is considered as having a $C_7$ aryl group.

The alkyl group as a substituent is the same as the alkyl group as described above as long as R is a $C_{1-5}$ alkyl group, a $C_{2-5}$ alkenyl group, or a $C_{1-12}$ aryl group. The number of carbon atoms in the alkyl group as a substituent is from 1 to 11, preferably from 1 to 6, and more preferably from 1 to 3.

The alkenyl group as a substituent is the same as the alkenyl group as described above as long as R is a $C_{1-5}$ alkyl group, a $C_{2-5}$ alkenyl group, or a $C_{1-12}$ aryl group. The number of carbon atoms in the alkenyl group as a substituent is from 2 to 11, preferably from 2 to 6, and more preferably from 2 to 3.

The aryl group as a substituent is the same as the aryl group as described above as long as R is a $C_{1-5}$ alkyl group, a $C_{2-5}$ alkenyl group, or a $C_{1-12}$ aryl group. The number of carbon atoms in the aryl group as a substituent is from 1 to 11, preferably from 3 to 9, and more preferably from 5 to 7.

The alkoxy group as a substituent is a $C_{1-11}$ alkoxy group, preferably a $C_{1-6}$ alkoxy group, and more preferably a $C_{1-3}$ alkoxy group, provided that R is a $C_{1-5}$ alkyl group, a $C_{2-5}$ alkenyl group, or a $C_{1-12}$ aryl group. Examples of the alkoxy group include a methoxy group, an ethoxy group, an n-propoxy group, an isopropoxy group, an n-butoxy group, an isobutoxy group, an s-butoxy group, a t-butoxy group, an n-pentoxy group, an isopentoxy group, and a phenoxy group.

The amino group as a substituent includes an amino group without a substituent on the nitrogen atom ($-NH_2$), and an amino group in which some or all of the hydrogen atoms bound to the nitrogen atom are substituted with carbon atoms. The number of carbon atoms in the amino group substituted with carbon atoms is from 1 to 11, preferably from 1 to 6, and more preferably from 1 to 3, provided that R is a $C_{1-5}$ alkyl group, a $C_{2-5}$ alkenyl group, or a $C_{1-12}$ aryl group. Examples of the amino group include a methylamino group, an ethylamino group, a propylamino group, a butylamino group, a dimethylamino group, a diethylamino group, an anilino group, a toluidino group, an anisidino group, and an N-methyl-N-phenylamino group.

Examples of the monovalent group containing a carbonyl group as a substituent include a formyl group, an acyl group, a carboxy group, an amide group, an alkoxy carbonyl group, a thiocarboxy group, and a thioester group.

The acyl group is a substituent in which a carbonyl group is linked with an alkyl group, an alkenyl group, or an aryl group. The total number of carbon atoms derived from a carbonyl group (one) and derived from an alkyl group, alkenyl group, or an aryl group is from 2 to 11, preferably from 2 to 7, and more preferably from 2 to 4, provided that R is a $C_{1-5}$ alkyl group, a $C_{2-5}$ alkenyl group, or a $C_{1-12}$ aryl group. Examples of the acyl group include an acetyl group, a propionyl group, a butylcarbonyl group, a vinylcarbonyl group, and a benzoyl group.

The amide group includes an amide group without a substituent on the nitrogen atom ($-CONH_2$), and an amide group in which some or all of the hydrogen atoms bound to the nitrogen atom are substituted with carbon atoms. The number of carbon atoms in the amide group, which is the total of the number of carbon atoms derived from a carbonyl group (one) and the number of carbon atoms substituted on the nitrogen atom, is from 1 to 11, preferably from 1 to 7, and more preferably from 1 to 4, provided that R is a $C_{1-5}$ alkyl group, a $C_{2-5}$ alkenyl group, or a $C_{1-12}$ aryl group. Examples of the amide group include an unsubstituted amide group, an N-methylamide group, an N-ethylamide group, an N-phenylamide group, an N,N-dimethylamide group, and an N-methyl-N-phenylamide group.

The alkoxy carbonyl group is a substituent in which a carbonyl group and an alkoxy group are linked, and also referred to as an ester group. The total number of carbon atoms derived from a carbonyl group (one) and derived from an alkoxy group is from 2 to 11, preferably from 2 to 7, and more preferably from 2 to 4, provided that R is a $C_{1-5}$ alkyl group, a $C_{2-5}$ alkenyl group, or a $C_{1-12}$ aryl group. Examples of the alkoxy carbonyl group include a methoxycarbonyl group, an ethoxycarbonyl group, a butoxycarbonyl group, and a phenoxycarbonyl group.

The thioester group is a substituent in which a carbonyl group and an alkylthio group or an arylthio group are linked. The total number of carbon atoms derived from a carbonyl group (one) and derived from an alkylthio group or an arylthio group is from 2 to 11, preferably from 2 to 7, and more preferably from 2 to 4, provided that R is a $C_{1-5}$ alkyl group, a $C_{2-5}$ alkenyl group, or a $C_{1-12}$ aryl group. Examples of the thioester group include a methylthiocarbonyl group, an ethylthiocarbonyl group, a butylthiocarbonyl group, and a phenylthiocarbonyl group.

The monovalent group containing a carbonyl group may be a substituent in which one or more hydrogen(s) in an alkyl group is/are substituted with a carbonyl group(s). Examples of such a substituent include a 2-acetoxyethyl group, a 2-acetoethyl group, and a 2-(acetoacetoxy)ethyl group.

The alkylthio group as a substituent is a $C_{1-11}$ alkylthio group, preferably a $C_{1-6}$ alkylthio group, and more preferably a $C_{1-3}$ alkylthio group, provided that R is a $C_{1-5}$ alkyl group, a $C_{2-5}$ alkenyl group, or a $C_{1-12}$ aryl group. Examples of the alkylthio group include a methylthio group, an ethylthio group, a propylthio group, and an isopropylthio group.

The arylthio group as a substituent is a $C_{1-11}$ arylthio group, preferably a $C_{3-9}$ arylthio group, and more preferably a $C_{5-7}$ arylthio group, provided that R is a $C_{1-5}$ alkyl group, a $C_{2-5}$ alkenyl group, or a $C_{1-12}$ aryl group. Examples of the arylthio group include a phenylthio group, and a tolylthio group.

Among compounds that satisfy the conditions described above, the component A is preferably methacrylonitrile, acetonitrile, propionitrile, acrylonitrile, benzonitrile, cyclohexanecarbonitrile, 3-hydroxypropionitrile, 3-methoxypropionitrile, 2-hydroxypropionitrile, 2-aminopropionitrile, 4-cyanophenol, 4-aminobenzonitrile, 4'-cyanoacetophenone, or 4-(methylthio)benzonitrile, more preferably methacrylonitrile, acetonitrile, propionitrile, acrylonitrile, benzonitrile, 3-hydroxypropionitrile, 3-methoxypropionitrile, 4-aminobenzonitrile, 4'-cyanoacetophenone, or 4-(methylthio)benzonitrile, and still more preferably methacrylonitrile, acetonitrile, propionitrile, acrylonitrile, benzonitrile, 3-hydroxypropionitrile, or 4-(methylthio)benzonitrile, from the viewpoint of quality stability of methyl methacrylate-containing composition during storage.

One or two or more component(s) A may be contained.

(Component B)

The methyl methacrylate-containing composition according to the present invention comprises a polymerization inhibitor (component B). As used herein, the term "polymerization inhibitor" means a compound having a function to inhibit the polymerization reaction of methyl methacrylate. Examples of the polymerization inhibitor include a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound. When the component B is included, the progress of the polymerization reaction of methyl methacrylate via a radical polymerization mechanism during storage of methyl methacrylate can be repressed. As described above, when the component A and the component B coexist, the generation of methyl methacrylate dimers, and the generation of methyl pyruvate caused by oxidation of methyl methacrylate can be efficiently repressed.

Examples of the polymerization inhibitor that is a phenol compound include alkylphenol, hydroxyphenol, aminophenol, nitrophenol, nitrosophenol, alkoxyphenol, and tocopherol.

Examples of the alkylphenol include o-cresol, m-cresol, p-cresol, 2-t-butyl-4-methylphenol, 2,4-dimethyl-6-t-butylphenol, 2,6-di-t-butyl-4-methylphenol, 2-t-butylphenol, 4-t-butylphenol, 2,4-di-t-butylphenol, 2-methyl-4-t-butylphenol, 4-t-butyl-2,6-dimethylphenol, 2,2'-methylene-bis(6-t-butyl-4-methylphenol), 2,2'-methylenebis(4-ethyl-6-t-butylphenol), 4,4'-thiobis(3-methyl-6-t-butylphenol), and 3,5-di-t-butyl-4-hydroxytoluene.

Examples of the hydroxyphenol include hydroquinone, 2-methylhydroquinone, 2-t-butylhydroquinone, 2,5-di-t-butylhydroquinone, 2,6-di-t-butylhydroquinone, 2,5-di-t-amylhydroquinone, 2-t-butylmethoxyhydroquinone, 2,3,5-trimethylhydroquinone, 2,5-dichlorohydroquinone, 1,2-dihydroxybenzene, 2-acetylhydroquinone, 4-methylcatechol, 4-t-butylcatechol, 2-methylresorcinol, 4-methylresorcinol, and 2,3-dihydroxyacetophenone.

Examples of the aminophenol include o-aminophenol, m-aminophenol, p-aminophenol, 2-(N,N-dimethylamino) phenol, and 4-(ethylamino)phenol.

Examples of the nitrophenol include o-nitrophenol, m-nitrophenol, p-nitrophenol, and 2,4-dinitrophenol.

Examples of the nitrosophenol include o-nitrosophenol, m-nitrosophenol, p-nitrosophenol, and $\alpha$-nitroso-$\beta$-naphthol.

Examples of the alkoxyphenol include 2-methoxyphenol, 2-ethoxyphenol, 2-isopropoxyphenol, 2-t-butoxyphenol, 4-methoxyphenol, 4-ethoxyphenol, 4-propoxyphenol, 4-butoxyphenol, 4-t-butoxyphenol, 4-heptoxyphenol, hydroquinone monobenzyl ether, t-butyl-4-methoxyphenol, dit-butyl-4-methoxyphenol, pyrogallol-1,2-dimethylether, and hydroquinone monobenzoate.

Examples of the tocopherol include $\alpha$-tocopherol, and 2,3-dihydro-2,2-dimethyl-7-hydroxybenzofuran.

Examples of the polymerization inhibitor that is a quinone compound include p-benzoquinone, chloro-p-benzoquinone, 2,5-dichloro-p-benzoquinone, 2,6-dichloro-p-benzoquinone, tetrachloro-p-benzoquinone, tetrabromo-p-benzoquinone, 2,3-dimethyl-p-benzoquinone, 2,5-dimethyl-p-benzoquinone, methoxy-p-benzoquinone, and methyl-p-benzoquinone.

Examples of the polymerization inhibitor that is a nitrobenzene compound include nitrobenzene, o-dinitrobenzene, m-dinitrobenzene, p-dinitrobenzene, 2,4-dinitrotoluene, dinitrodurene, and 2,2-diphenyl-1-picrylhydrazyl.

Examples of the polymerization inhibitor that is an N-oxyl compound include 4-hydroxy-2,2,6,6-tetramethyl-piperidine-N-oxyl, 4-oxo-2,2,6,6-tetramethyl-piperidine-N-oxyl, 4-acetoxy-2,2,6,6-tetramethyl-piperidine-N-oxyl, 2,2,6,6-tetramethyl-piperidine-N-oxyl, piperidine-1-oxyl, 4-(dimethylamino)-2,2,6,6-tetramethyl-piperidine-N-oxyl, 4-amino-2,2,6,6-tetramethyl-piperidine-N-oxyl, 4-ethanoloxy-2,2,6,6-tetramethyl-piperidine-N-oxyl, 4-benzoyloxy-2,2,6,6-tetramethylpiperidine-N-oxyl, 2,2,5,5-tetramethyl-piperidine-N-oxyl, 3-amino-2,2,5,5-tetramethyl-piperidine-N-oxyl, 4,4',4''-tris(2,2,6,6-tetramethyl-piperidine-N-oxyl)phosphite, 3-oxo-2,2,5,5-tetramethylpyrrolidine-N-oxyl, pyrrolidine-1-oxyl, 2,2,5,5-tetramethyl-1-oxa-3-azacyclopentyl-3-oxy, 2,2,5,5-tetramethyl-3-pyrrolinyl-1-oxy-3-carboxylic acid, 2,2,3,3,5,5,6,6-octamethyl-1,4-diazacyclohexyl-1,4-dioxy, di-tert-butylnitroxide, and di-tert-amylnitroxide.

Examples of the polymerization inhibitor that is an amine compound include N,N-diphenylamine, alkylated diphenylamine, 4,4'-dicumyl-diphenylamine, 4,4'-dioctyldiphenylamine, 4-aminodiphenylamine, p-nitrosodiphenylamine, N-nitrosodinaphthylamine, N-nitrosodiphenylamine, N-nitrosophenylnaphthylamine, N-nitrosophenylhydroxylamine, N,N'-dialkyl-p-phenylenediamine (the alkyl groups may be the same or different, and may each independently comprise 1 to 4 carbon atoms, and may be linear or branched), N,N'-diphenyl-p-phenylenediamine, N-phenyl-N'-isopropyl-p-phenylenediamine, N-(1,3-dimethylbutyl)-N'-phenyl-1,4-phenylenediamine, N,N'-di-2-naphthyl-p-phenylenediamine, N,N-diethylhydroxylamine, 1,4-benzenediamine, N-(1,4-dimethylpentyl)-N'-phenyl-1,4-benzenediamine, N-(1,3-dimethylbutyl)-N'-phenyl-1,4-benzenediamine, 6-ethoxy-2,2,4-trimethyl-1,2-dihydroquinoline, 2,2,4-trimethyl-1,2-dihydroquinoline polymer, aldol-$\alpha$-naphthylamine, N-phenyl-$\beta$-naphthylamine, 4-hydroxy-2,2,6,6-tetramethylpiperidine, 4-benzoyloxy-2,2,6,6-tetramethylpiperidine, 1,4-dihydroxy-2,2,6,6-tetramethylpiperidine, and 1-hydroxy-4-benzoyloxy-2,2,6,6-tetramethylpiperidine.

Examples of the polymerization inhibitor that is a phosphorus-containing compound include triphenylphosphine, triphenyl phosphite, triethyl phosphite, tris(isodecyl) phosphite, tris(tridecyl) phosphite, phenyl diisooctyl phosphite, phenyl diisodecyl phosphite, phenyl di(tridecyl) phosphite, diphenyl isooctyl phosphite, diphenyl isodecyl phosphite, diphenyl tridecyl phosphite, phosphonic acid [1,1-diphenyl-4,4'-diylbistetrakis-2,4-bis(1,1-dimethylethyl)phenyl] ester, triphenyl phosphite, tris(nonylphenyl) phosphite, 4,4'-isopropylidenediphenol alkyl phosphite, tris(2,4-di-tert-butylphenyl) phosphite, tris(biphenyl) phosphite, distearyl pentaerythritol diphosphite, di(2,4-di-tert-butylphenyl) pentaerythritol diphosphate, di(nonylphenyl) pentaerythritol diphosphite, phenyl bisphenol-A pentaerythritol diphosphite, tetra(tridecyl)-4,4'-butylidenebis(3-methyl-6-tertbutylphenol)diphosphite, hexa(tridecyl)-1,1,3-tris(2-methyl-4-hydroxy-5-tert-butylphenyl) butanetriphosphite, 3,5-di-tert-butyl-4-hydroxybenzyl phosphate diethyl ester, sodium-bis(4-tert-butylphenyl) phosphate, sodium-2,2'-methylene -bis(4,6-di-tert-butylphenyl)phosphate, and 1,3-bis(diphenoxyphosphonyloxy)benzene.

Examples of the polymerization inhibitor that is a sulfur-containing compound include diphenyl sulfide, phenothiazine, 3-oxophenothiazine, 5-oxophenothiazine, phenothiazine dimer, 1,4-dimercaptobenzene, 1,2-dimercaptobenzene, 2-mercaptophenol, 4-mercaptophenol, 2-(methylthio)phenol, 3,7-bis(dimethylamino)phenothiazinium chloride, and sulfur (simple substance).

Examples of the polymerization inhibitor that is an iron-containing compound include iron (III) chloride.

Examples of the polymerization inhibitor that is a copper-containing compound include copper dimethyldithiocarbamate, copper diethyldithiocarbamate, copper dibutyldithiocarbamate, copper salicylate, copper acetate, copper thiocyanate, copper nitrate, copper chloride, copper carbonate, copper hydroxide, copper acrylate, and copper methacrylate.

Examples of the polymerization inhibitor that is a manganese-containing compound include manganese dialkyl dithiocarbamate (the alkyl groups are each any of a methyl group, an ethyl group, a propyl group, and a butyl group, and may be the same or different), manganese diphenyl dithiocarbamate, manganese formate, manganese acetate, manganese octanoate, manganese naphthenate, manganese permanganate, and ethylenediaminetetraacetic acid manganese salt.

Among those described above, the component B is preferably at least one polymerization inhibitor(s) selected from the group consisting of a phenol compound, an N-oxyl compound, an amine compound, a phosphorus-containing compound, and a sulfur-containing compound, and more preferably at least one polymerization inhibitor(s) selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine, from the viewpoint of quality stability of the methyl methacrylate-containing composition during storage.

One or two or more component(s) B may be contained.

In cases where the methyl methacrylate-containing composition comprises a compound that corresponds to both components A and B, the compound is considered as the component B. Thus, the methyl methacrylate-containing composition must comprise a component A other than the compound. In cases where two or more compounds that correspond to both components A and B are comprised, the compound with the highest molar concentration in the methyl methacrylate composition is considered as the component B, and the other compound(s) is/are considered as the component(s) A.

(Concentrations of Component A and Component B)

When the concentration of a component A is MA ($\mu$mol/L) and the concentration of a component B is MB ($\mu$mol/L), MB/MA is preferably 0.003 or more from the viewpoint of efficiency in reducing the generation of methyl methacrylate dimers and methyl pyruvate. The upper limit of MB/MA is not particularly limited, and is usually 100 or less, and preferably 50 or less.

MA is preferably from 1 to 20,000 $\mu$mol/L. When MA is 1 $\mu$mol/L or more, the effect of reducing the generation of methyl methacrylate dimers and methyl pyruvate can be sufficiently obtained. When MA is 20,000 $\mu$mol/L or less, the impurity content after production of methyl methacrylate polymer by polymerization of the methyl methacrylate-containing composition according to the present invention is reduced, so that adverse effects on physical properties of the polymer can be prevented. The lower limit of MA is more preferably 10 $\mu$mol/L or more, still more preferably 50 $\mu$mol/L or more, and particularly preferably 60 $\mu$mol/L or more. The upper limit of the concentration of the component A is more preferably 15,000 $\mu$mol/L or less, still more preferably 10,000 $\mu$mol/L or less, particularly preferably 5,000 $\mu$mol/L or less, and most preferably 1,000 $\mu$mol/L or less.

MB is preferably from 1 to 5,000 $\mu$mol/L. When MB is 1 $\mu$mol/L or more, the effect of reducing the generation of methyl methacrylate dimers and methyl pyruvate can be sufficiently obtained. When MB is 5,000 $\mu$mol/L or less, the impurity content after production of methyl methacrylate polymer by polymerization of the methyl methacrylate-containing composition according to the present invention is reduced, so that adverse effects on physical properties of the polymer can be prevented. The lower limit of MB is more preferably 10 $\mu$mol/L or more, and still more preferably 30 $\mu$mol/L or more. The upper limit of the concentration of the component B is more preferably 2,500 $\mu$mol/L or less, still more preferably 2,000 $\mu$mol/L or less, particularly preferably 1,000 $\mu$mol/L or less, and most preferably 600 $\mu$mol/L or less.

(Concentration of Methyl Methacrylate)

The concentration of methyl methacrylate in the methyl methacrylate-containing composition according to the present invention is from 99 to 99.99% by mass. When the concentration of methyl methacrylate is 99% by mass or more, the impurity content after production of methyl methacrylate polymer by polymerization of the methyl methacrylate-containing composition according to the present invention is reduced, so that adverse effects on physical properties of the polymer can be prevented. When the concentration of methyl methacrylate is 99.99% by mass or less, the purification cost can be reduced. The lower limit of the concentration of methyl methacrylate is preferably 99.8% by mass or more.

(Component C)

The methyl methacrylate-containing composition according to the present invention may contain other compound (component C) as long as the concentration of methyl methacrylate of from 99 to 99.99% by mass is satisfied. Examples of the component C include, impurities generated during the production of methyl methacrylate.

For example, methyl methacrylate may contain diacetyl as an impurity. From the viewpoint of reducing coloration of the methyl methacrylate-containing composition, the concentration of diacetyl is preferably 55 $\mu$mol/L, more preferably 20 $\mu$mol/L or less, still more preferably 10 $\mu$mol/L or less, and particularly preferably 1 $\mu$mol/L.

(Analysis of Methyl Methacrylate-containing Composition)

Inclusion of a component A, a component B, a component C, and water in the methyl methacrylate-containing composition can be determined, for example, by GC-MS measurement. When the GC-MS chart of the methyl methacrylate-containing composition has a peak at the same retention time as a reference material for the component A, and when the m/z value detected in the mass spectrum of the peak corresponds to the exact mass of the component A, it can be determined that the methyl methacrylate-containing composition comprises the component A. In cases where the reference material of the component A is not available, it can be determined that the peak is the component A peak when the mass spectrum peak pattern shown in the GC-MS chart of the methyl methacrylate-containing composition corresponds to the mass spectrum pattern of the component A recorded in the mass spectrum database. In other words, it can be determined that the methyl methacrylate-containing composition comprises the component A. Examples of the mass spectrum database include NIST20, NIST17, NIST14, and NIST14s. When detection by GC-MS measurement is impossible due to low volatility, LC-MS can be used for detection. Inclusion of a component B, a component C, and water can also be determined by the same method.

The concentration of methyl methacrylate can be determined, for example, by carrying out GC-FID measurement of the methyl methacrylate-containing composition, quantifying methyl methacrylate using the area normalization method, and correcting the resulting value using the water concentration determined with a Karl-Fisher moisture meter. The concentration of the component A can be determined, for example, by carrying out GC measurement of the methyl methacrylate-containing composition and using the internal standard method. When the reference material of the component A is not available, and thus cannot be quantified by the internal standard method, GC-FID measurement for any organic compound having known concentration is carried out under the same conditions as the methyl methacrylate-containing composition, and then the concentration of the component A can be calculated using the following equation:

$$\text{Concentration of component A} = \frac{N}{N_A} \times \frac{S_A}{S} \times M$$

In the equation, N is the number of carbon atoms that the organic compound having known concentration contains in one molecule; $N_A$ is the number of carbon atoms that the component A contains in one molecule; $S_A$ is the peak area of the component A, S is the peak area of the organic compound having known concentration, and M is the concentration ($\mu$mol/L) of the organic compound having known concentration.

When quantification by GC measurement is impossible due to low volatility, a chromatography method such as LC can be used for quantification.

The concentrations of the component B and component C can be also determined by the same method as the component A described above.

Inclusion of water by the methyl methacrylate-containing composition and its concentration can be determined by the Karl-Fischer method.

[Method of Producing Methyl Methacrylate-containing Composition]

The method of producing the methyl methacrylate-containing composition according to the present invention may be a method in which a component A and a component B are added to methyl methacrylate. Methyl methacrylate to be used may be a commercially available product, or methyl methacrylate produced by known methods such as the acetone cyanohydrin (ACH) method, the new acetone cyanohydrin (new ACH) method, the C4 direct oxidation method, the direct methyl esterification method, the ethylene method, and the new ethylene method may be used. The component A and component B to be used may be commercially available products, or those synthesized by known methods may be used. When methyl methacrylate produced by a known method such as the acetone cyanohydrin (ACH) method, the new acetone cyanohydrin (new ACH) method, the C4 direct oxidation method, the direct methyl esterification method, the ethylene method, or the new ethylene method is used, the component A or the component B may be added to raw materials or in the course of the production process to produce the methyl methacrylate-containing composition. In cases where a component A or a component B is produced as a by-product during the methyl methacrylate production process, a part of the component A or the component B produced may be left for production of the methyl methacrylate-containing composition.

[Methods of Evaluating Storage Stability and Heat Stability]

The methyl methacrylate-containing composition according to the present invention has high quality stability during storage. The method of evaluating the quality stability of the methyl methacrylate-containing composition during storage may be, for example, a method in which the methyl methacrylate-containing composition is actually stored for a long period, and the amounts of methyl methacrylate dimers and methyl pyruvate generated are determined. From the viewpoint of work simplicity, a method may be used in which the methyl methacrylate-containing composition is heated for a short time, and the amounts of methyl methacrylate dimers and methyl pyruvate generated are determined. In cases of heating for short time, the heating temperature is preferably from 50 to 100°C, and the heating time period is preferably from 1 to 24 hours. In the present invention, the quality stability of the methyl methacrylate-containing composition during storage is evaluated based on the amounts of methyl methacrylate dimers and methyl pyruvate generated when the methyl methacrylate-containing composition is stored at 25°C for 14 days.

[Method of Producing Methyl Methacrylate Polymer]

The method of producing methyl methacrylate polymer according to the present invention comprises a step of polymerizing a polymeric composition comprising the methyl methacrylate-containing composition according to the present invention.

<Polymeric Composition>

The polymeric composition may comprise, as needed, a monomer that can be copolymerized with methyl methacrylate, and other additives.

(Monomer that can be Copolymerized with Methyl Methacrylate)

Examples of the monomer that can be copolymerized with methyl methacrylate include the following:

a methacrylate ester, such as ethyl methacrylate, iso-propyl methacrylate, n-butyl methacrylate, isobutyl methacrylate, tert-butyl methacrylate, 2-ethylhexyl methacrylate, phenyl methacrylate, or benzyl methacrylate;
an acrylate ester, such as methyl acrylate, ethyl acrylate, n-butyl acrylate, iso-butyl acrylate, tert-butyl acrylate, or 2-ethylhexyl acrylate;
an unsaturated carboxylic acid, such as acrylic acid, methacrylic acid, maleic acid, or itaconic acid;
an unsaturated carboxylic anhydride, such as maleic anhydride, or itaconic anhydride;
maleimide, such as N-phenylmaleimide, or N-cyclohexylmaleimide;
a vinyl monomer containing a hydroxy group, such as 2-hydroxyethyl acrylate, 2-hydroxyethyl methacrylate, or 2-hydroxypropyl methacrylate;
a vinyl ester, such as vinyl acetate, or vinyl benzoate;
vinyl chloride, vinylidene chloride, and derivatives thereof;
a vinyl monomer containing nitrogen, such as methacrylamide, or acrylonitrile;
a monomer containing an epoxy group, such as glycidyl acrylate, or glycidyl methacrylate;
an aromatic vinyl monomer, such as styrene, or $\alpha$-methylstyrene;
alkane diol di(meth)acrylate, such as ethylene glycol di(meth)acrylate, 1,2-propylene glycol di(meth)acrylate, 1,3-butyleneglycol di(meth)acrylate, or 1,6-hexanediol di(meth)acrylate;
polyoxyalkylene glycol di(meth)acrylate, such as diethylene glycol di(meth)acrylate, dipropylene glycol di(meth)acrylate, triethylene glycol (meth)acrylate, tetraethylene glycol di(meth)acrylate, polyethylene glycol di(meth)acrylate, or neopentyl glycol di(meth)acrylate;
a vinyl monomer having two or more ethylenic unsaturated bonds in its molecule, such as divinylbenzene;
an unsaturated polyester prepolymer obtained from at least one polyvalent carboxylic acid including ethylenic unsaturated polycarboxylic acid and at least one diol; and
a vinyl ester prepolymer obtained by acrylic modification of an end of an epoxy group.

Among those described above, the monomer that can be copolymerized with methyl methacrylate is preferably at least one selected from the group consisting of the methacrylate esters and the acrylate esters. This enables polymerization of the polymeric composition to obtain a methyl methacrylate polymer with excellent balance of transparency, heat resistance, and moldability. The monomer that can be copolymerized with methyl methacrylate is more preferably an acrylate ester, and particularly preferably at least one selected from the group consisting of methyl acrylate, ethyl acrylate, and n-butyl acrylate.

One or two or more monomer(s) that can be copolymerized with methyl methacrylate may be used. When the component A is a monomer that can be copolymerized with methyl methacrylate, the component A may be used as the monomer that can be copolymerized with methyl methacrylate, or the monomer that can be copolymerized with methyl methacrylate apart from the component A may be used.

The content of the monomer that can be copolymerized with methyl methacrylate contained in the polymeric composition is preferably from 0 to 50 parts by mass with respect to 100 parts by mass of methyl methacrylate. This enables obtaining a methyl methacrylate polymer with high transparency. The upper limit of the content of the monomer that can be copolymerized with methyl methacrylate with respect to 100 parts by mass of methyl methacrylate is more preferably 40 parts by mass or less, and still more preferably 30 parts by mass or less. The lower limit of the content of the monomer that can be copolymerized with methyl methacrylate with respect to 100 parts by mass of methyl methacrylate is more preferably 0.01 parts by mass or more, still more preferably 0.1 parts by mass or more, and particularly preferably 1 part by mass or more.

(Other Additives)

Other additives preferably include a polymerization initiator. Other additives may also include, for example, a chain transfer agent, a mold release agent, a lubricant, a plasticizer, an antioxidant, an antistatic agent, a light stabilizer, an ultraviolet absorber, a flame retardant, a flame retardant promoter, a polymerization inhibitor, a filler, a pigment, a dye, a silane coupling agent, a leveling agent, an antifoam, and a fluorescent agent, as needed. One or two or more other additive(s) may be contained.

Examples of the polymerization initiator include the following:

an azo compound, such as 2,2'-azobisisobutyronitrile, 2,2'-azobis(2-methylbutyronitrile), 2,2'-azobis(2-methylpropionitrile), 2,2'-azobis(2,4-dimethylvaleronitrile), 2,2'-azobis(2,4,4-trimethylpentane), 2-2'-azobis(2-methylpropane), 1,1-azobis(cyclohexanecarbonitrile), and dimethyl-2,2'-azobisisobutyrate;
an organic peroxide, such as benzoyl peroxide, 2,5-dimethyl-2,5-bis(t-butylperoxy)hexane, 1,1-bis(t-butylperoxy) cyclohexane, 1,1-bis(t-butylperoxy)-3,5,5-trimethylcyclohexane, t-butyl peroxy-2-ethylhexanoate, t-butyl peroxyisobutyrate, t-butyl peroxybenzoate, t-hexyl peroxybenzoate, t-butyl peroxyisopropyl monocarbonate, t-butyl peroxy-3,5,5-trimethyl hexanoate, t-butyl peroxylaurate, t-butyl peroxyacetate, t-hexyl peroxyisopropyl monocarbonate, t-hexyl peroxy-2-ethyl hexanoate, t-amyl peroxy-2-ethyl hexanoate, 1,1,3,3-tetramethyl butyl peroxyethyl hexanoate, 1,1,2-trimethyl propyl peroxy-2-ethyl hexanoate, 1,1,3,3-tetramethyl butyl peroxyisopropyl monocarbonate, 1,1,2-trimethyl propyl peroxyisopropyl monocarbonate, 1,1,3,3-tetramethyl butyl peroxyisononanoate, 1,1,2-trimethyl propyl peroxyisononanoate, di-t-butyl peroxide, di-t-hexyl peroxide, lauroyl peroxide, and dilauroyl peroxide;
a persulfate compound, such as potassium persulfate; and
a redox polymerization initiator.

Among those described above, the polymerization initiator is preferably at least one selected from the group consisting of the azo compound and the organic peroxide from the viewpoint of storage stability and reactivity with methyl methacrylate.

The amount of the polymerization initiator used is preferably from 0.0001 to 1 part by mass with respect to 100 parts by mass of the total of methyl methacrylate and the monomer that can be copolymerized with methyl methacrylate.

<Method of Polymerizing Polymeric Composition>

Examples of the polymerization method include a bulk polymerization method, a solution polymerization method, an emulsion polymerization method, and a suspension polymerization method. From the viewpoint of environmental load due to the use of solvents and the like, and of transparency of the methyl methacrylate polymer to be obtained, a bulk polymerization method is preferable.

Specific means for the bulk polymerization method is not particularly limited, and a known casting polymerization method such as a cell casting method or a continuous casting method can be used for production.

The casting polymerization method is a method for obtaining methyl methacrylate polymer by injecting a polymeric composition into a mold composed of two inorganic glass plates or metal plates (for example, SUS plates) placed opposite each other at a predetermined distance with the periphery sealed with a gasket such as a soft resin tube, and allowing the composition to polymerize.

The mold for casting polymerization is not particularly limited, and known molds can be used. Examples of the mold for cell casting include those in which two plate-like bodies, such as inorganic glass plates, chromium-plated metal plates, and stainless steel plates, are placed opposite each other at a predetermined distance, and a gasket is placed around the periphery to allow the plate-like bodies and the gasket to form a sealed space. Examples of the mold for continuous casting include those in which a sealed space is formed by opposing faces of a pair of endless belts running in the same direction at the same speed, and gaskets running at the same speed as the endless belt on both sides of the endless belt.

The spacing of the void in a mold is adjusted as appropriate to obtain a resin sheet with a desired thickness, and is generally from 1 to 30 mm.

The polymerization temperature is preferably from 125 to 210°C. This enables achieving an appropriate polymerization rate. More preferably, the lower limit of the polymerization temperature is 130°C or more, and the upper limit is 180°C or less. The polymerization time is not particularly limited, and can be, for example, from 0.5 to 24 hours.

EXAMPLES

The present invention is described in detail below with Examples and Comparative Examples, but is not limited to these Examples. Unless otherwise stated, the terms "%" and "ppm" in the examples and the comparative examples mean "% by weight" and "ppm by weight," respectively.

The water concentration contained in a methyl methacrylate reagent was determined by the Karl-Fischer method. The composition of the components of the methyl methacrylate-containing composition before being stored was determined based on the amounts of the raw materials added. Methyl methacrylate dimers and methyl pyruvate in the methyl methacrylate-containing composition after being stored were determined by an absolute calibration curve method using GC-MS. The measurement conditions for GC-MS are shown below.

Instrument: GC-MS measuring system (product name: QP-2010SE, manufactured by Shimadzu Corporation)

[Conditions for GC]

Column (product name: DB-WAX, manufactured by Agilent Technologies, Inc.);
Length: 60 m, Inner diameter: 0.32 mm, Film thickness: 1.00 $\mu$m;
Injection volume: 1.0 $\mu$L;
Vaporizing chamber temperature: 210°C;
Column oven temperature: held at 35°C for 10 minutes, raised from 35°C to 150°C at 5 °C/min, held at 150°C for 17 minutes, raised from 150°C to 220°C at 5 °C/min, and held at 220°C for 6 minutes;
Carrier gas: helium;
Injection mode: split (split ratio: 50);
Control mode: constant linear velocity (25.0 cm/sec);
Pressure: 26.1 KPa;
Total flow: 52.5 mL/min;
Purge flow: 3.0 mL/min; and
Column flow: 0.97 mL/min.

[Conditions for MS]

Ionization method: EI (Electron Ionization);
Ion source temperature: 250°C;
Interface temperature: 250°C;
m/z detection range: 10 to 300; and
Detection time: 70 minutes.

Determination of the water concentration by the Karl-Fischer method was carried out using automated water measurement equipment (product name: AQV-2200, manufactured by Hiranuma Co., Ltd.).

(Example 1)

Using methacrylonitrile as a component A, 0.0229 g of the component A was added to 10.0275 g of a methyl methacrylate reagent (water concentration: 240 ppm) to prepare a methyl methacrylate solution (A-1 solution). The concentration of the component A in the A-1 solution is shown in Table 1.

Using 2,4-dimethyl-6-t-butylphenol as a component B, 0.0228 g of the component B was added to 10.0026 g of a methyl methacrylate reagent (water concentration: 240 ppm) to prepare a methyl methacrylate solution (B-1 solution). The concentration of the component B in the B-1 solution is shown in Table 1.

Next, 0.10016 g of the A-1 solution and 0.1027 g of the B-1 solution were added to 20.0053 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored at 25°C for 14 days. The amounts of methyl methacrylate dimers and methyl pyruvate generated in the methyl methacrylate-containing composition after the storage are shown in Table 2.

(Examples 2 to 5)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Examples 6 to 10)

A-1 solutions were prepared in the same manner as in Example 1 except that a compound shown in Table 1 was used as a component A, and the amounts of the methyl methacrylate reagent and the component A were changed as shown in Table 1.

B-1 solutions were prepared in the same manner as in Example 1 except that the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Examples 11 to 17)

A-1 solutions were prepared in the same manner as in Example 1.

B-1 solutions were prepared in the same manner as in Example 1 except that a compound shown in Table 1 was used as a component B, and the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Example 18)

An A-1 solution and a B-1 solution were prepared in the same manner as in Example 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Example 19)

An A-1 solution was prepared in the same manner as in Example 1.

A B-1 solution was prepared in the same manner as in Example 1 except that the amounts of the methyl methacrylate reagent and the component B were changed as shown in Table 1.

Next, a methyl methacrylate-containing composition was prepared in the same manner as in Example 1 except that the amounts of the methyl methacrylate reagent, the A-1 solution, and the B-1 solution were changed as shown in Table 2. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Example 20)

A B-1 solution was prepared in the same manner as in Example 1.

Next, using methacrylonitrile as a component A, 0.0229 g of the component A and 0.1090 g of the B-1 solution were added to 20.0100 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Examples 21 and 22)

A-1 solutions were prepared in the same manner as in Example 1.

B-1 solutions were prepared in the same manner as in Example 1 except that the amounts of the methyl methacrylate reagent and the component B were changed as shown in Table 1.

Next, methyl methacrylate-containing compositions were prepared in the same manner as in Example 1 except that the amounts of the methyl methacrylate reagent, the A-1 solution, and the B-1 solution were changed as shown in Table 2. The concentrations of the components in the methyl methacrylate-containing compositions are shown in Table 2.

The obtained methyl methacrylate-containing compositions were stored as in Example 1. The amounts of methyl methacrylate dimers and methyl pyruvate generated in the methyl methacrylate-containing compositions after the storage are shown in Table 2.

(Comparative Example 1)

A B-1 solution was prepared in the same manner as in Example 1.

Next, 0.2213 g of the B-1 solution was added to 40.0273 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 2)

40.0000 g of a methyl methacrylate reagent (water concentration: 240 ppm) was used as a methyl methacrylate-containing composition and stored as in Example 1. The amounts of methyl methacrylate dimers and methyl pyruvate generated in the methyl methacrylate-containing composition after the storage are shown in Table 2.

(Comparative Example 3)

A B-1 solution was prepared in the same manner as in Example 1.

Using diacetyl as a component C, 0.0205 g of the component C was added to 9.9913 g of a methyl methacrylate reagent (water concentration: 240 ppm) to prepare a methyl methacrylate solution (C-1 solution). The concentration of the component C in the C-1 solution is shown in Table 1.

Next, 0.2151 g of the B-1 solution and 0.2069 g of the C-1 solution were added to 40.0218 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 4)

An A-1 solution was prepared in the same manner as in Example 1.

Next, 0.2054 g of the A-1 solution was added to 40.0249 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

(Comparative Example 5)

An A-1 solution was prepared in the same manner as in Example 1.

A B-1 solution was prepared in the same manner as in Example 1 except that the amounts of the methyl methacrylate reagent and the component B were changed as shown in Table 1.

Next, 0.1010 g of the A-1 solution, 0.1051 g of the B-1 solution, and 0.3003 g of pure water were added to 20.0192 g of a methyl methacrylate reagent (water concentration: 240 ppm) to prepare a methyl methacrylate-containing composition. The concentrations of the components in the methyl methacrylate-containing composition are shown in Table 2.

The obtained methyl methacrylate-containing composition was stored as in Example 1. The amounts of methyl methacrylate dimers and methyl pyruvate generated in the methyl methacrylate-containing composition after the storage are shown in Table 2.

Table 1

| | Amount of MMA regent added [g] | A-1 solution — Component A | | | Amount of MMA regent added [g] | B-1 solution — Component B | | | Amount of MMA regent added [g] | C-1 solution — Component C | | |
|---|---|---|---|---|---|---|---|---|---|---|---|---|
| | | Compound name | Added amount [g] | Concentration [ppm] | | Compound name | Added amount [g] | Concentration [ppm] | | Compound name | Added amount [g] | Concentration [ppm] |
| Example 1 | 10.0275 | Methacrylonitrile | 0.0229 | 2279 | 10.0026 | 2,4-Dimethyl-6-t-butylphenol | 0.0228 | 2274 | - | - | - | - |
| Example 2 | 10.0093 | Acetonitrile | 0.0203 | 2024 | 10.0026 | 2,4-Dimethyl-6-t-butylphenol | 0.0228 | 2274 | - | - | - | - |
| Example 3 | 10.0437 | Propionitrile | 0.0235 | 2334 | 10.0026 | 2,4-Dimethyl-6-t-butylphenol | 0.0228 | 2274 | - | - | - | - |
| Example 4 | 10.0030 | Acrylonitrile | 0.0206 | 2055 | 10.0026 | 2,4-Dimethyl-6-t-butylphenol | 0.0228 | 2274 | - | - | - | - |
| Example 5 | 10.0112 | Benzonitrile | 0.0249 | 2481 | 10.0026 | 2,4-Dimethyl-6-t-butylphenol | 0.0228 | 2274 | - | - | - | - |
| Example 6 | 10.0212 | 3-Hydroxypropionitrile | 0.0259 | 2578 | 40.0080 | 2,4-Dimethyl-6-t-butylphenol | 0.0828 | 2065 | - | - | - | - |
| Example 7 | 10.0068 | 3-Methoxypropionitrile | 0.0215 | 2144 | 40.0080 | 2,4-Dimethyl-6-t-butylphenol | 0.0828 | 2065 | - | - | - | - |
| Example 8 | 10.0952 | 4-Aminobenzonitrile | 0.0214 | 2115 | 40.0080 | 2,4-Dimethyl-6-t-butylphenol | 0.0828 | 2065 | - | - | - | - |
| Example 9 | 10.0125 | 4'-Cyanoacetophenone | 0.0208 | 2073 | 40.0080 | 2,4-Dimethyl-6-t-butylphenol | 0.0828 | 2065 | - | - | - | - |
| Example 10 | 10.0050 | 4-(Methylthio)benzonitrile | 0.0201 | 2005 | 40.0080 | 2,4-Dimethyl-6-t-butylphenol | 0.0828 | 2065 | - | - | - | - |
| Example 11 | 10.0275 | Methacrylonitrile | 0.0229 | 2279 | 10.0051 | 4-Methoxyphenol | 0.0191 | 1905 | - | - | - | - |
| Example 12 | 10.0275 | Methacrylonitrile | 0.0229 | 2279 | 10.0238 | Hydroquinone | 0.0233 | 2319 | - | - | - | - |
| Example 13 | 10.0275 | Methacrylonitrile | 0.0229 | 2279 | 10.0204 | Phenothiazine | 0.0238 | 2370 | - | - | - | - |
| Example 14 | 10.0275 | Methacrylonitrile | 0.0229 | 2279 | 10.0021 | 4-Hydroxy-2,2,6,6-tetramethylpiperidine-N-oxyl | 0.0202 | 2016 | - | - | - | - |
| Example 15 | 10.0275 | Methacrylonitrile | 0.0229 | 2279 | 10.1244 | N,N-Diphenylamine | 0.0222 | 2188 | - | - | - | - |
| Example 16 | 10.0275 | Methacrylonitrile | 0.0229 | 2279 | 10.0077 | N-Nitrosodiphenylamine | 0.0208 | 2074 | - | - | - | - |

(continued)

| | A-1 solution | | | | B-1 solution | | | | C-1 solution | | | |
|---|---|---|---|---|---|---|---|---|---|---|---|---|
| | Amount of MMA regent added [g] | Component A | | | Amount of MMA regent added [g] | Component B | | | Amount of MMA regent added [g] | Component C | | |
| | | Compound name | Added amount [g] | Concentration [ppm] | | Compound name | Added amount [g] | Concentration [ppm] | | Compound name | Added amount [g] | Concentration [ppm] |
| Example 17 | 10.0275 | Methacrylonitrile | 0.0229 | 2279 | 10.0061 | Triphenyl phosphite | 0.0223 | 2224 | - | - | - | - |
| Example 18 | 10.0275 | Methacrylonitrile | 0.0229 | 2279 | 10.0026 | 2,4-Dimethyl-6-t-butylphenol | 0.0228 | 2274 | - | - | - | - |
| Example 19 | 10.0275 | Methacrylonitrile | 0.0229 | 2279 | 40.0221 | 2,4-Dimethyl-6-t-butylphenol | 0.0829 | 2067 | - | - | - | - |
| Example 20 | - | - | - | - | 10.0026 | 2,4-Dimethyl-6-t-butylphenol | 0.0228 | 2274 | - | - | - | - |
| Example 21 | 10.0275 | Methacrylonitrile | 0.0229 | 2279 | 40.0221 | 2,4-Dimethyl-6-t-butylphenol | 0.0829 | 2067 | - | - | - | - |
| Example 22 | 10.0275 | Methacrylonitrile | 0.0229 | 2279 | 40.0221 | 2,4-Dimethyl-6-t-butylphenol | 0.0829 | 2067 | - | - | - | - |
| Comparative example 1 | - | - | - | - | 10.0026 | 2,4-Dimethyl-6-t-butylphenol | 0.0228 | 2274 | - | - | - | - |
| Comparative example 2 | - | - | - | - | - | - | - | - | - | - | - | - |
| Comparative example 3 | - | - | - | - | 10.0026 | 2,4-Dimethyl-6-t-butylphenol | 0.0228 | 2274 | 9.9913 | Diacetyl | 0.0205 | 2048 |
| Comparative example 4 | 10.0275 | Methacrylonitrile | 0.0229 | 2279 | - | - | - | - | - | - | - | - |
| Comparative example 5 | 10.0275 | Methacrylonitrile | 0.0229 | 2279 | 40.0221 | 2,4-Dimethyl-6-t-butylphenol | 0.0829 | 2067 | - | - | - | - |

Table 2

| | Added amount [g] | | | | | | MMA-containing composition | | | | | | | | Amount of MMA dimer generated [ppm] | Amount of methyl pyruvate generated [ppm] |
|---|---|---|---|---|---|---|---|---|---|---|---|---|---|---|---|---|
| | | | | | | | MMA | Component A | | Component B | | Component C | | | | |
| | MMA reagent | A-1 solution | B-1 solution | C-1 solution | Component A | Pure water | Concentration [%] | Compound name | Concentration MA [μmol/L] | Compound name | Concentration MB [μmol/L] | Compound name | Concentration [μmol/L] | MB/MA | | |
| Example 1 | 20.0053 | 0.1016 | 0.1027 | - | - | - | 99.97 | Methacrylonitrile | 161 | 2,4-Dimethyl-6-t-butylphenol | 61 | - | - | 0.380 | 19 | 2 |
| Example 2 | 20.0274 | 0.1012 | 0.1020 | - | - | - | 99.97 | Acetonitrile | 233 | 2,4-Dimethyl-6-t-butylphenol | 61 | - | - | 0.261 | 20 | 2 |
| Example 3 | 20.0157 | 0.1048 | 0.1025 | - | - | - | 99.97 | Propionitrile | 207 | 2,4-Dimethyl-6-t-butylphenol | 61 | - | - | 0.294 | 22 | 4 |
| Example 4 | 20.0108 | 0.1048 | 0.1022 | - | - | - | 99.97 | Acrylonitrile | 190 | 2,4-Dimethyl-6-t-butylphenol | 61 | - | - | 0.321 | 22 | 3 |
| Example 5 | 20.0116 | 0.1030 | 0.1055 | - | - | - | 99.97 | Benzonitrile | 116 | 2,4-Dimethyl-6-t-butylphenol | 63 | - | - | 0.543 | 20 | 1 |
| Example 6 | 10.0048 | 0.0536 | 0.0509 | - | - | - | 99.97 | 3-Hydroxypropionitrile | 182 | 2,4-Dimethyl-6-t-butylphenol | 55 | - | - | 0.303 | 22 | 1 |
| Example 7 | 10.0194 | 0.0525 | 0.0513 | - | - | - | 99.97 | 3-Methoxypropionitrile | 123 | 2,4-Dimethyl-6-t-butylphenol | 55 | - | - | 0.449 | 24 | 4 |
| Example 8 | 10.0091 | 0.0508 | 0.0528 | - | - | - | 99.97 | 4-Aminobenzonitrile | 85 | 2,4-Dimethyl-6-t-butylphenol | 57 | - | - | 0.672 | 24 | 4 |
| Example 9 | 10.0197 | 0.0520 | 0.0521 | - | - | - | 99.97 | 4'-Cyanoacetophenone | 69 | 2,4-Dimethyl-6-t-butylphenol | 56 | - | - | 0.813 | 26 | 5 |
| Example 10 | 10.0065 | 0.0530 | 0.0527 | - | - | - | 99.97 | 4-(Methylthio)benzonitrile | 66 | 2,4-Dimethyl-6-t-butylphenol | 57 | - | - | 0.857 | 13 | 2 |
| Example 11 | 20.0078 | 0.1046 | 0.1031 | - | - | - | 99.97 | Methacrylonitrile | 166 | 4-Methoxyphenol | 74 | - | - | 0.445 | 16 | 2 |
| Example 12 | 20.0117 | 0.1054 | 0.1103 | - | - | - | 99.97 | Methacrylonitrile | 167 | Hydroquinone | 108 | - | - | 0.649 | 23 | 0 |
| Example 13 | 20.0017 | 0.1024 | 0.1042 | - | - | - | 99.97 | Methacrylonitrile | 162 | Phenothiazine | 58 | - | - | 0.356 | 14 | 0 |
| Example 14 | 10.0102 | 0.0524 | 0.0520 | - | - | - | 99.97 | Methacrylonitrile | 166 | 4-Hydroxy-2,2,6,6-tetramethylpiperidine -N-oxyl | 57 | - | - | 0.342 | 7 | 11 |

(continued)

| | Added amount [g] | | | | | | MMA-containing composition | | | | | | | | Amount of MMA dimer generated [ppm] | Amount of methyl pyruvate generated [ppm] |
|---|---|---|---|---|---|---|---|---|---|---|---|---|---|---|---|---|
| | | | | | | | MMA | Component A | | Component B | | Component C | | | | |
| | MMA reagent | A-1 solution | B-1 solution | C-1 solution | Compo nent A | Pure water | Concent ration [%] | Compound name | Concent ration MA [μmol/L] | Compound name | Concent ration MB [μmol/L] | Compound name | Concent ration [μmol/L] | MB/MA | | |
| Example 15 | 10.0056 | 0.0550 | 0.0512 | - | - | - | 99.97 | Methacrylonitrile | 174 | N,N-Diphenylamine | 62 | - | - | 0.354 | 8 | 0 |
| Example 16 | 10.0100 | 0.0535 | 0.0505 | - | - | - | 99.97 | Methacrylonitrile | 170 | N-Nitrosodiphenylamine | 49 | - | - | 0.291 | 3 | 0 |
| Example 17 | 10.0026 | 0.0509 | 0.0555 | - | - | - | 99.97 | Methacrylonitrile | 161 | Triphenyl phosphite | 37 | - | - | 0.230 | 0 | 23 |
| Example 18 | 19.0088 | 1.0032 | 0.1049 | - | - | - | 99.96 | Methacrylonitrile | 1599 | 2,4-Dimethyl-6-t-butylphenol | 63 | - | - | 0.039 | 20 | 2 |
| Example 19 | 8.0156 | 2.0075 | 0.0509 | - | - | - | 99.93 | Methacrylonitrile | 6389 | 2,4-Dimethyl-6-t-butylphenol | 55 | - | - | 0.009 | 18 | 0 |
| Example 20 | 20.0100 | - | 0.1090 | - | 0.0229 | - | 99.86 | Methacrylonitrile | 15997 | 2,4-Dimethyl-6-t-butylphenol | 65 | - | - | 0.004 | 20 | 0 |
| Example 21 | 19.0239 | 0.1033 | 1.0040 | - | - | - | 99.96 | Methacrylonitrile | 165 | 2,4-Dimethyl-6-t-butylphenol | 546 | - | - | 3.318 | 19 | 0 |
| Example 22 | 7.5411 | 0.0513 | 2.5057 | - | - | - | 99.92 | Methacrylonitrile | 163 | 2,4-Dimethyl-6-t-butylphenol | 2716 | - | - | 16.675 | 23 | 0 |
| Comparative example 1 | 40.0273 | - | 0.2213 | - | - | - | 99.97 | - | - | 2,4-Dimethyl-6-t-butyl-phenol | 66 | - | - | - | 29 | 2 |
| Compara-tive example 2 | 40.0000 | - | - | - | - | - | 99.98 | - | - | - | - | - | - | - | 0 | 25 |
| Comparative example 3 | 40.0218 | - | 0.2151 | 0.2069 | - | - | 99.97 | - | - | 2,4-Dimethyl-6-t-butylphenol | 64 | Diacetyl | 115 | - | 0 | 1056 |
| Comparative example 4 | 40.0249 | 0.2054 | - | - | - | - | 99.97 | Methacrylonitrile | 164 | - | - | - | - | - | 33 | 11 |

(continued)

| | Added amount [g] | | | | | | MMA-containing composition | | | | | | | | MB/MA | Amount of MMA dimer generated [ppm] | Amount of methyl pyruvate generated [ppm] |
|---|---|---|---|---|---|---|---|---|---|---|---|---|---|---|---|---|---|
| | | | | | | | MMA | Component A | | Component B | | Component C | | | | | |
| | MMA reagent | A-1 solution | B-1 solution | C-1 solution | Component A | Pure water | Concentration [%] | Compound name | Concentration MA [$\mu$mol/L] | Compound name | Concentration MB [$\mu$mol/L] | Compound name | Concentration [$\mu$mol/L] | | | | |
| Comparative example 5 | 20.0192 | 0.1010 | 0.1051 | - | - | 0.3003 | 98.51 | Methacrylonitrile | 158 | 2,4-Dimethyl-6-t-butylphenol | 56 | - | - | 0.355 | 30 | 4 |

INDUSTRIAL APPLICABILITY

According to the present invention, methyl methacrylate-containing compositions that can be used, for example, as raw materials for acrylic resins can be stored stably for a long period of time, which is industrially useful.

**Claims**

1.  A methyl methacrylate-containing composition, comprising:

    methyl methacrylate;
    a nitrile compound represented by the following Formula (1) (component A); and
    a polymerization inhibitor (component B),
    wherein the concentration of methyl methacrylate is from 99 to 99.99% by mass,

    $$R\text{-}C\equiv N \qquad (1)$$

    wherein, in Formula (1) above, R represents a $C_{1-5}$ chain or cyclic alkyl group, a $C_{2-5}$ chain or cyclic alkenyl group, or a $C_{1-12}$ aryl or heteroaryl group, which may further have a substituent.

2.  The methyl methacrylate-containing composition according to claim 1, wherein when the concentration of the component A is MA ($\mu$mol/L), and the concentration of the component B is MB ($\mu$mol/L), MB/MA is 0.003 or more.

3.  The methyl methacrylate-containing composition according to claim 1 or 2, wherein when the concentration of the component A is MA ($\mu$mol/L), MA is from 1 to 20,000 $\mu$mol/L.

4.  The methyl methacrylate-containing composition according to claim 3, wherein the MA is from 10 to 15,000 $\mu$mol/L.

5.  The methyl methacrylate-containing composition according to any one of claims 1 to 4, wherein when the concentration of the component B is MB ($\mu$mol/L), MB is from 1 to 5,000 $\mu$mol/L.

6.  The methyl methacrylate-containing composition according to claim 5, wherein the MB is from 10 to 2,500 $\mu$mol/L.

7.  The methyl methacrylate-containing composition according to any one of claims 1 to 6, wherein the molecular weight of the component A is 1,000 or less.

8.  The methyl methacrylate-containing composition according to any one of claims 1 to 7, wherein the component B is at least one polymerization inhibitor selected from the group consisting of a phenol compound, a quinone compound, a nitrobenzene compound, an N-oxyl compound, an amine compound, a phosphorus-containing compound, a sulfur-containing compound, an iron-containing compound, a copper-containing compound, and a manganese-containing compound.

9.  The methyl methacrylate-containing composition according to any one of claims 1 to 8, wherein the component B is at least one polymerization inhibitor selected from the group consisting of a phenol compound, an N-oxyl compound, an amine compound, a phosphorus-containing compound, and a sulfur-containing compound.

10. The methyl methacrylate-containing composition according to any one of claims 1 to 9, wherein the component B is at least one polymerization inhibitor selected from the group consisting of hydroquinone, 4-methoxyphenol, 2,4-dimethyl-6-t-butylphenol, 2,6-di-t-butyl-4-methylphenol, 4-hydroxy-2,2,6,6-tetramethylpiperidine-N-oxyl, N,N-diphenylamine, N-nitrosodiphenylamine, triphenyl phosphite, and phenothiazine.

11. The methyl methacrylate-containing composition according to any one of claims 1 to 10, wherein the concentration of methyl methacrylate is from 99.8 to 99.99% by mass.

12. The methyl methacrylate-containing composition according to any one of claims 1 to 11, wherein the composition does not contain diacetyl, or the concentration of diacetyl contained is 55 $\mu$mol/L or less.

13. The methyl methacrylate-containing composition according to any one of claims 1 to 12, wherein in the Formula (1), R

is a $C_{1-3}$ chain or cyclic alkyl group, a $C_{2-3}$ chain or cyclic alkenyl group, or a $C_{6-8}$ aryl or heteroaryl group.

14. The methyl methacrylate-containing composition according to any one of claims 1 to 13, wherein in the Formula (1), R is a methyl group, an ethyl group, a vinyl group, an isopropenyl group, or a phenyl group.

15. A method of producing a methyl methacrylate polymer, comprising a step of polymerizing a polymeric composition comprising the methyl methacrylate-containing composition according to any one of claims 1 to 14.

16. The method of producing a methyl methacrylate polymer according to claim 15, wherein the polymeric composition comprises a monomer that can be copolymerized with methyl methacrylate.

**Patentansprüche**

1. Methylmethacrylathaltige Zusammensetzung, umfassend:

Methylmethacrylat,
eine durch die folgende Formel (1) dargestellte Nitrilverbindung (Komponente A) und
einen Polymerisationsinhibitor (Komponente B),
wobei die Konzentration von Methylmethacrylat 99 bis 99,99 Masse-% beträgt,

$$R\text{-}C\equiv N \qquad (1)$$

wobei in der obigen Formel (1) R eine kettenförmige oder cyclische $C_{1-5}$-Alkylgruppe, eine kettenförmige oder cyclische $C_{2-5}$-Alkenylgruppe oder eine $C_{1-12}$-Aryl- oder Heteroarylgruppe darstellt, die ferner einen Substituenten aufweisen können.

2. Methylmethacrylathaltige Zusammensetzung gemäß Anspruch 1, wobei, wenn die Konzentration der Komponente A MA (μmol/L) ist und die Konzentration der Komponente B MB (μmol/L) ist, MB/MA 0,003 oder mehr beträgt.

3. Methylmethacrylathaltige Zusammensetzung gemäß Anspruch 1 oder 2, wobei, wenn die Konzentration der Komponente A MA (μmol/L) ist, MA 1 bis 20.000 μmol/L beträgt.

4. Methylmethacrylathaltige Zusammensetzung gemäß Anspruch 3, wobei MA 10 bis 15.000 μmol/L beträgt.

5. Methylmethacrylathaltige Zusammensetzung gemäß einem der Ansprüche 1 bis 4, wobei, wenn die Konzentration der Komponente B MB (μmol/L) ist, MB 1 bis 5.000 μmol/L beträgt.

6. Methylmethacrylathaltige Zusammensetzung gemäß Anspruch 5, wobei MB 10 bis 2.500 μmol/L beträgt.

7. Methylmethacrylathaltige Zusammensetzung gemäß einem der Ansprüche 1 bis 6, wobei das Molekulargewicht der Komponente A 1.000 oder weniger beträgt.

8. Methylmethacrylathaltige Zusammensetzung gemäß einem der Ansprüche 1 bis 7, wobei die Komponente B mindestens ein Polymerisationsinhibitor, ausgewählt aus der Gruppe, bestehend aus einer Phenolverbindung, einer Chinonverbindung, einer Nitrobenzolverbindung, einer N-Oxylverbindung, einer Aminverbindung, einer phosphorhaltigen Verbindung, einer schwefelhaltigen Verbindung, einer eisenhaltigen Verbindung, einer kupferhaltigen Verbindung und einer manganhaltigen Verbindung, ist.

9. Methylmethacrylathaltige Zusammensetzung gemäß einem der Ansprüche 1 bis 8, wobei die Komponente B mindestens ein Polymerisationsinhibitor, ausgewählt aus der Gruppe, bestehend aus einer Phenolverbindung, einer N-Oxylverbindung, einer Aminverbindung, einer phosphorhaltigen Verbindung und einer schwefelhaltigen Verbindung, ist.

10. Methylmethacrylathaltige Zusammensetzung gemäß einem der Ansprüche 1 bis 9, wobei die Komponente B mindestens ein Polymerisationsinhibitor, ausgewählt aus der Gruppe, bestehend aus Hydrochinon, 4-Methoxyphenol, 2,4-Dimethyl-6-t-butylphenol, 2,6-Di-t-butyl-4-methylphenol, 4-Hydroxy-2,2,6,6-tetramethylpiperidin-N-oxyl, N,N-Diphenylamin, N-Nitrosodiphenylamin, Triphenylphosphit und Phenothiazin, ist.

**11.** Methylmethacrylathaltige Zusammensetzung gemäß einem der Ansprüche 1 bis 10, wobei die Konzentration des Methylmethacrylats 99,8 bis 99,99 Masse-% beträgt.

**12.** Methylmethacrylathaltige Zusammensetzung gemäß einem der Ansprüche 1 bis 11, wobei die Zusammensetzung kein Diacetyl enthält, wobei die Konzentration von enthaltenem Diacetyl 55 μmol/L oder weniger beträgt.

**13.** Methylmethacrylathaltige Zusammensetzung gemäß einem der Ansprüche 1 bis 12, wobei in der Formel (1) R eine kettenförmige oder cyclische $C_{1-3}$-Alkylgruppe, eine kettenförmige oder cyclische $C_{2-3}$-Alkenylgruppe oder eine $C_{6-8}$-Aryl- oder Heteroarylgruppe ist.

**14.** Methylmethacrylathaltige Zusammensetzung gemäß einem der Ansprüche 1 bis 13, wobei in der Formel (1) R eine Methylgruppe, eine Ethylgruppe, eine Vinylgruppe, eine Isopropenylgruppe oder eine Phenylgruppe ist.

**15.** Verfahren zur Herstellung eines Methylmethacrylatpolymers, umfassend einen Schritt zum Polymerisieren einer polymeren Zusammensetzung, umfassend die methylmethacrylathaltige Zusammensetzung gemäß einem der Ansprüche 1 bis 14.

**16.** Verfahren zur Herstellung eines Methylmethacrylatpolymers gemäß Anspruch 15, wobei die polymere Zusammensetzung ein Monomer umfasst, das mit Methylmethacrylat copolymerisiert werden kann.

## Revendications

**1.** Composition à teneur en méthacrylate de méthyle,
comprenant :

du méthacrylate de méthyle ;
un composé nitrile représenté par la Formule (1) suivante (composant A) ; et,
un inhibiteur de polymérisation (composant B),
dans laquelle la concentration en méthacrylate de méthyle est comprise entre 99 et 99,99 % en masse,

$$R-C=N \qquad (1)$$

dans laquelle, dans la Formule (1) ci-dessus, R représente un groupe alkyle à chaîne ou cyclique en $C_{1-5}$, un groupe alcényle à chaîne ou cyclique en $C_{2-5}$, ou un groupe aryle ou hétéroaryle en $C_{1-12}$, qui peut en outre comporter un substituant.

**2.** Composition à teneur en méthacrylate de méthyle selon la revendication 1, dans laquelle lorsque la concentration en composant A est MA (μmol/L), et la concentration en composant B est MB (μmol/L), MB/MA est supérieure ou égale à 0,003.

**3.** Composition à teneur en méthacrylate de méthyle selon la revendication 1 ou la revendication 2, dans laquelle lorsque la concentration en composant A est MA (μmol/L), MA est comprise entre 1 et 20 000 μmol/L.

**4.** Composition à teneur en méthacrylate de méthyle selon la revendication 3, dans laquelle la MA est comprise entre 10 et 15 000 μmol/L.

**5.** Composition à teneur en méthacrylate de méthyle selon l'une quelconque des revendications 1 à 4, dans laquelle lorsque la concentration en composant B est MB (μmol/L), MB est comprise entre 1 et 5 000 μmol/L.

**6.** Composition à teneur en méthacrylate de méthyle selon la revendication 5, dans laquelle la MB est comprise entre 10 et 2 500 μmol/L.

**7.** Composition à teneur en méthacrylate de méthyle selon l'une quelconque des revendications 1 à 6, dans laquelle le poids moléculaire du composant A est inférieur ou égal à 1 000.

**8.** Composition à teneur en méthacrylate de méthyle selon l'une quelconque des revendications 1 à 7, dans laquelle le composant B est au moins un inhibiteur de polymérisation choisi dans le groupe constitué par un composé phénolique, un composé quinone, un composé nitrobenzène, un composé N-oxyle, un composé amine, un composé contenant du phosphore, un composé contenant du soufre, un composé contenant du fer, un composé contenant du cuivre et un composé contenant du manganèse.

**9.** Composition à teneur en méthacrylate de méthyle selon l'une quelconque des revendications 1 à 8, dans laquelle le composant B est au moins un inhibiteur de polymérisation choisi dans le groupe constitué par un composé phénolique, un composé N-oxyle, un composé amine, un composé contenant du phosphore et un composé contenant du soufre.

**10.** Composition à teneur en méthacrylate de méthyle selon l'une quelconque des revendications 1 à 9, dans laquelle le composant B est au moins un inhibiteur de polymérisation choisi dans le groupe constitué par l'hydroquinone, le 4-méthoxyphénol, le 2,4-diméthyl-6-t-butylphénol, le 2,6-di-t-butyl-4-méthylphénol, 4-hydroxy-2,2,6,6-tétraméthylpipéridine-N-oxyle, la N,N-diphénylamine, la N-nitrosodiphénylamine, le phosphite de triphényle et la phénothiazine.

**11.** Composition à teneur en méthacrylate de méthyle selon l'une quelconque des revendications 1 à 10, dans laquelle la concentration en méthacrylate de méthyle est comprise entre 99,8 et 99,99 % en masse.

**12.** Composition à teneur en méthacrylate de méthyle selon l'une quelconque des revendications 1 à 11, dans laquelle la composition ne contient pas de diacétyle, ou la concentration en diacétyle contenue est comprise entre 55 $\mu$mol/L ou moins.

**13.** Composition à teneur en méthacrylate de méthyle selon l'une quelconque des revendications 1 à 12, dans laquelle dans la Formule (1), R est un groupe alkyle à chaîne ou cyclique en $C_{1-3}$, un groupe alcényle à chaîne ou cyclique en $C_{2-3}$, ou un groupe aryle ou hétéroaryle en $C_{6-8}$.

**14.** Composition à teneur en méthacrylate de méthyle selon l'une quelconque des revendications 1 à 13, dans laquelle dans la Formule (1), R est un groupe méthyle, un groupe éthyle, un groupe vinyle, un groupe isopropényle ou un groupe phényle.

**15.** Procédé de production d'un polymère de méthacrylate de méthyle, comprenant une étape de polymérisation d'une composition polymère comprenant la composition à teneur en méthacrylate de méthyle selon l'une quelconque des revendications 1 à 14.

**16.** Procédé de production d'un polymère de méthacrylate de méthyle selon la revendication 15, dans lequel la composition polymère comprend un monomère qui peut être copolymérisé avec le méthacrylate de méthyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004155757 A
- JP 2005502695 A
- JP H10504553 A
- JP 2002533309 A
- JP 2002513034 A
- US 5877344 A

**Non-patent literature cited in the description**

- Development of Catalyst for Producing Methyl Methacrylate. **TORU KURODA**. Catalysts & Catalysis. Catalysis Society of Japan, 2003, vol. 45, 366-371
- On the Functions of Polymerization Inhibitors. **TAKAYUKI OTSU**. Synthetic Organic Chemistry. The Society of Synthetic Organic Chemistry, 1975, vol. 33, 634-640